# EUROPEAN PATENT APPLICATION

(11) **EP 1 938 822 A1**
(43) Date of publication of application: **02.07.2008**
(21) Application number: 06126840.5
(22) Date of filing: 21.12.2006
(51) Int. Cl.: A61K 31/4704, A61K 9/12, A61P 11/00, A61K 31/352, A61K 31/407, A61K 31/4706, A61K 31/431, A61K 31/65, A61K 31/085, A61K 31/546

(54) **Combination therapy for the treatment of airways disease**

(71) Applicant: Novartis AG, 4056 Basel (CH)
(72) Inventor: The designation of the inventor has not yet been filed
(74) Representative: McLean, Craig Sutherland

(57) **Abstract**

A medicament that comprises, separately or together (A) a compound of formula 1 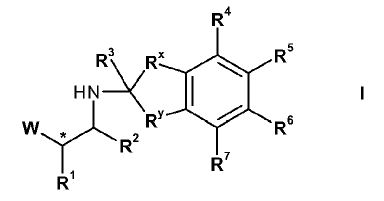 in free or salt or solvate form, wherein W, R^{x}, R^{y}, R¹, R², R³, R⁴, R⁵, R⁶ and R⁷ have the meanings used herein; and (B) an antibacterial agent; for simultaneous, sequential or separate administration in the treatment of an inflammatory, infective or obstructive airways disease.

## Description

This invention relates to organic compounds and their use as pharmaceuticals, in particular for the treatment of inflammatory, infective or obstructive airways diseases.

In a first aspect, the present invention provides a medicament comprising, separately or together
(A) a compound of formula I 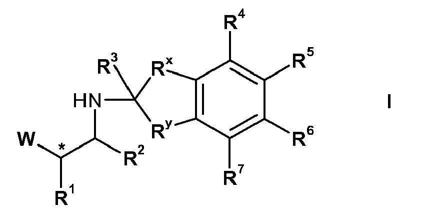 in free or salt or solvate form, wherein
   W is a group of formula 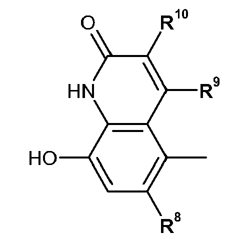 R^{x} and R^{y} are both -CH₂- or -(CH₂)₂-;
   R¹ is hydrogen, hydroxy, or C₁-C₁₀-alkoxy;
   R² and R³ are each independently hydrogen or C₁-C₁₀-alkyl;
   R⁴, R⁵, R⁶ and R⁷ are each independently hydrogen, halogen, cyano, hydroxy,
   C₁-C₁₀-alkoxy, C₆-C₁₀-aryl, C₁-C₁₀-alkyl, C₁-C₁₀-alkyl substituted by one or more halogen atoms or one or more hydroxy or C₁-C₁₀-alkoxy groups, C₁-C₁₀-alkyl interrupted by one or more hetero atoms, C₂-C₁₀-alkenyl, trialkylsilyl, carboxy,
   C₁-C₁₀-alkoxycarbonyl, or -CONR¹¹R¹² where R¹¹ and R¹² are each independently hydrogen or C₁-C₁₀-alkyl,
   or R⁴ and R⁵, R⁵ and R⁶, or R⁶ and R⁷ together with the carbon atoms to which they are attached denote a 5-, 6- or 7-membered carbocyclic ring or a 4- to 10-membered heterocyclic ring; and
   R⁸, R⁹ and R¹⁰ are each independently hydrogen or C₁-C₄-alkyl; and
(B) an antibacterial agent;
   for simultaneous, sequential or separate administration in the treatment of an inflammatory, infective or obstructive airways disease.

Compounds of formula I in free or salt or solvate form possess beta-2 adrenoceptor agonist activity. They commonly have a rapid onset of action and have a prolonged stimulating action on the β₂-adrenoceptor, for example up 24 hours or longer. They may be prepared by using the procedures described in international patent applications WO 2000/75114, WO 2003/76387, WO 2004/76422 or WO 2004/87668.

The choice of a suitable antibacterial agent, however, will depend on the nature and level of the disease state. Thus, for cystic fibrosis and bronchiectasis, an antibacterial agent effective against *Pseudomonas aeruginosa* and/or *Burkholderia cepacia* will be highly desirable. For chronic bronchitis, an antibacterial agent effective particularly against *Hemophilus influenzae* is desirable.

It has now surprisingly been found that a significant unexpected therapeutic benefit, particularly a synergistic therapeutic benefit, in the treatment of inflammatory, infective or obstructive airways diseases can be obtained by combination therapy using a compound of formula I in free or salt or solvate form, and an antibacterial agent. For instance, it is possible using this combination therapy to reduce the dosages of one or both of the active ingredients required for a given therapeutic effect considerably compared with those required using treatment with the active ingredients alone, thereby minimising possibly undesirable side effects. In particular, it has been found that these combinations, particularly compositions containing 5-[2-(5,6-diethyl-indan-2-ylamino)-1-hydroxy-ethyl]-8-hydroxy-1H-quinolin-2-one maleate and an antibacterial agent, induce an anti-inflammatory activity which is significantly greater than that induced by 5-[2-(5,6-diethyl-indan-2-ylamino)-1-hydroxy-ethyl]-8-hydroxy-1H-quinolin-2-one maleate and an antibacterial agent alone. By dilating the airways 5-[2-(5,6-diethyl-indan-2-ylamino)-1-hydroxy-ethyl]-8-hydroxy-1H-quinolin-2-one maleate improves delivery to the affected area thus permitting lower dosing.

Furthermore, using the combination therapy of the invention, particularly using compositions containing 5-[2-(5,6-diethyl-indan-2-ylamino)-1-hydroxy-ethyl]-8-hydroxy-1H-quinolin-2-one maleate and an antibacterial agent, medicaments which have a rapid onset of action and a long duration of action may be prepared. Moreover, using such combination therapy, medicaments which result in a significant improvement in lung function may be prepared. Using the combination therapy of the invention, medicaments which provide improved control of inflammatory, infective or obstructive airways diseases, or a reduction in exacerbations of such diseases, may be prepared.

In a second aspect, the present invention provides a pharmaceutical composition comprising a mixture of effective amounts of (A) as hereinbefore defined, and (B) as hereinbefore defined, optionally together with at least one pharmaceutically acceptable carrier.

Preferably the pharmaceutical composition is a nebulisable composition or a dry powder.

In a third aspect, the present invention provides a method of treating an inflammatory, infective or obstructive airways disease which comprises administering to a subject in need of such treatment effective amounts of (A) as hereinbefore defined, and (B) as hereinbefore defined.

Preferably the inflammatory, infective or obstructive airways disease is asthma, chronic obstructive pulmonary disease (COPD) or cystic fibrosis.

The invention further provides the use of (A) as hereinbefore defined, and (B) as hereinbefore defined, in the preparation of a medicament for combination therapy by simultaneous, sequential or separate administration of (A) and (B) in the treatment of an inflammatory, infective or obstructive airways disease.

Terms used in the specification have the following meanings:
"C₁-C₁₀-alkoxy" as used herein denotes straight chain or branched alkoxy that contains 1 to 10 carbon atoms.
"C₁-C₁₀-alkyl" as used herein denotes straight chain or branched alkyl that contains one to ten carbon atoms.
"Halogen" or "halo" as used herein denotes an element belonging to group 17 (formerly group VII) of the Periodic Table of Elements, e.g. fluorine, chlorine, bromine or iodine.
"C₆-C₁₀-aryl" as used herein denotes a monovalent carbocyclic aromatic group that contains 6 to 10 carbon atoms and which may be, for example, a monocyclic group such as phenyl or a bicyclic group such as naphthyl.
"C₂-C₁₀-alkenyl" as used herein denotes straight chain or branched hydrocarbon chains that contain two to ten carbon atoms and one or more carbon-carbon double bonds.
"C₁-C₁₀-alkoxycarbonyl" as used herein denotes C₁-C₁₀-alkoxy as hereinbefore defined linked through an oxygen atom thereof to a carbonyl group.
"5-, 6 or 7-membered carbocyclic ring" as used herein denotes a carbocyclic group having 5 to 7 ring carbon atoms, either cycloaliphatic, such as a C₅-C₇-cycloalkyl, or aromatic, such as phenyl, which can be substituted by one or more, usually one or two, C₁-C₄-alkyl groups. Where C₃-C₇-cycloalkyl" denotes cycloalkyl having 3 to 7 ring carbon atoms, for example a monocyclic group such as a cyclopropyl, cyclobutyl, cyclopentyl, cyclohexyl or cycloheptyl, any of which can be substituted by one or more, usually one or two, C₁-C₄-alkyl groups, or a bicyclic group such as bicycloheptyl.
"4- to 10-membered heterocyclic ring having at least one ring nitrogen, oxygen or sulphur atom" as used herein may be, for example, pyrrole, pyrrolidine, pyrazole, imidazole, triazole, tetrazole, thiadiazole, oxazole, isoxazole, thiophene, thiazole, isothiazole, oxadiazole, pyridine, pyrazine, pyridazine, pyrimidine, piperidine, piperazine, triazine, oxazine, morpholino, quinoline, isoquinoline, naphthyridine, indane or indene.

Compounds of formula I in free or salt or solvate form possess beta-2 adrenoceptor agonist activity. They commonly have a rapid onset of action and have a prolonged stimulating action on the β₂-adrenoceptor, for example up 24 hours or longer.

Preferred compounds of formula I include those wherein R⁸, R⁹ and R¹⁰ are each H, R¹ is OH, R² and R³ are each H and
(i) R^{x} and R^{y} are both -CH₂-, and R⁴ and R⁷ are each CH₃O- and R⁵ and R⁶ are each H;
(ii) R^{x} and R^{y} are both -CH₂-, and R⁴ and R⁷ are each H and R⁵ and R⁶ are each CH₃CH₂-;
(iii) R^{x} and R^{y} are both -CH₂-, and R⁴ and R⁷ are each H and R⁵ and R⁶ are each CH₃-;
(iv) R^{x} and R^{y} are both -CH₂-, and R⁴ and R⁷ are each CH₃CH₂- and R⁵ and R⁶ are each H;
(v) R^{x} and R^{y} are both -CH₂-, and R⁴ and R⁷ are each H and R⁵ and R⁶ together denote -(CH₂)₄-;
(vi) R^{x} and R^{y} are both -CH₂-, and R⁴ and R⁷ are each H and R⁵ and R⁶ together denote -O(CH₂)₂O-;
(vii) R^{x} and R^{y} are both -CH₂-, and R⁴ and R⁷ are each H and R⁵ and R⁶ are each CH₃(CH₂)₃-;
(viii) R^{x} and R^{y} are both -CH₂-, and R⁴ and R⁷ are each H and R⁵ and R⁶ are each CH₃(CH₂)₂-;
(ix) R^{x} and R^{y} are both -(CH₂)₂-, R⁴, R⁵, R⁶ and R⁷ are each H; or
(x) R^{x} and R^{y} are both -CH₂-, and R⁴ and R⁷ are each H and R⁵ and R⁶ are each CH₃OCH₂-.

Especially preferred compounds of formula I include 8-hydroxy-5-[1-hydroxy-2-(indan-2-ylamino)-ethyl]-1H-quinolin-2-one, 5-[2-(5,6-dimethoxy-indan-2-ylamino)-1-hydroxy-ethyl]-8-hydroxy-1H-quinolin-2-one, 5-[2-(5,6-diethyl-indan-2-ylamino)-1-hydroxy-ethyl]-8-hydroxy-3-methyl-1H-quinolin-2-one, 5-[2-(5,6-diethyl-indan-2-ylamino)-1-hydroxy-ethyl]-8-methoxymethoxy-6-methyl-1H-quinolin-2-one, 5-[2-(5,6-diethyl-indan-2-ylamino)-1-hydroxy-ethyl]-8-hydroxy-6-methyl-1H-quinolin-2-one, 8-hydroxy-5-[2-(5,6-diethyl-indan-2-ylamino)-1-hydroxy-ethyl]-3,4-dihydro-1H-quinolin-2-one, 5-[(R)-2-(5,6-diethyl-2-methyl-indan-2-ylamino)-1-hydroxy-ethyl]-8-hydroxy-1H-quinolin-2-one, (S)-5-[2-(4,7-diethyl-indan-2-ylamino)-1-hydroxy-ethyl]-8-hydroxy-1H-quinolin-2-one hydrochloride, 5-[(R)-1-hydroxy-2-(6,7,8,9-tetrahydro-5H-benzocyclohepten-7-ylamino)-ethyl]-8-hydroxy-1H-quinolin-2-one hydrochloride, (R)-5-[2-(5,6-diethyl-indan-2-ylamino)-1-hydroxy-ethyl]-8-hydroxy-1H-quinolin-2-one maleate, (R)-5-[2-(5,6-diethyl-indan-2-ylamino)-1-hydroxy-ethyl]-8-hydroxy-1H-quinolin-2-one hydrochloride, (R)-8-hydroxy-5-[(S)-1-hydroxy-2-(4,5,6,7-tetramethyl-indan-2-ylamino)-ethyl]-1H-quinolin-2-one, 8-hydroxy-5-[(R)-1-hydroxy-2-(2-methyl-indan-2-ylamino)-ethyl]-1H-quinolin-2-one, 5-[2-(5,6-diethyl-indan-2-ylamino)-ethyl]-8-hydroxy-1H-quinolin-2-one, 8-hydroxy-5-[(R)-1-hydroxy-2-(2-methyl-2,3,5,6,7,8-hexahydro-1H-cyclopenta[b]naphthalen-2-ylamino)-ethyl]-1H-quinolin-2-one and 5-[(S)-2-(2,3,5,6,7,8-hexahydro-1H-cyclopenta[b]naph-thalen-2-ylamino)-1-hydroxy-ethyl]-8-hydroxy-1H-quinolin-2-one.

A particularly preferred compound of formula I is a compound of formula II 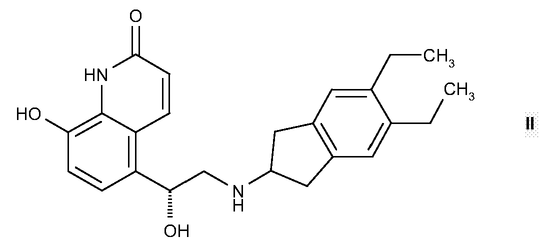 in free or pharmaceutically acceptable salt or solvate form, especially the maleate salt, namely (R)-5-[2-(5,6-diethyl-indan-2-ylamino)-1-hydroxyethyl]-8-hydroxy-1H-quinolin-2-one maleate.

Compounds of formula I in free or salt or solvate form may be prepared by using the procedures described in international patent applications WO 2000/75114, WO 2003/76387, WO 2004/76422 or WO 2004/87668, the contents of which are incorporated herein by reference.

Compounds of formula I include all pharmaceutically acceptable isotopically-labelled compounds of formula I wherein one or more atoms are replaced by atoms having the same atomic number, but an atomic mass or mass number different from the atomic mass or mass number usually found in nature. Examples of isotopes suitable for inclusion in the compounds of the invention include isotopes of hydrogen e.g. ²H and ³H, carbon e.g. ¹¹C, ¹³C and ¹⁴C, chlorine e.g. ³⁶Cl, fluorine e.g. ¹⁸F, iodine e.g. ¹²³I and ¹²⁵I, nitrogen e.g. ¹³N and ¹⁵N, oxygen e.g. ¹⁵O, ¹⁷O and ¹⁸O, and sulfur e.g. ³⁵S.

Certain isotopically-labelled compounds of formula I, for example those incorporating a radioactive isotope, are useful in drug and/or substrate tissue distribution studies. The radioactive isotopes tritium (³H) and carbon-14 (¹⁴C) are particularly useful for this purpose in view of their ease of incorporation and ready means of detection. Substitution with heavier isotopes such as deuterium (²H) may afford certain therapeutic advantages that result from greater metabolic stability, for example increased *in vivo* half-life or reduced dosage requirements, and hence may be preferred in some circumstances. Substitution with positron emitting isotopes, such as ¹¹C, ¹⁸F, ¹⁵O, and ¹³N can be useful in Positron Emission Tomography (PET) studies for examining substrate receptor occupancy.

Isotopically-labelled compounds of formula I can generally be prepared by conventional techniques known to those skilled in the art or by processes analogous to those described in the accompanying examples using an appropriate isotopically-labelled reagent in place of the non-labelled reagent previously used.

Compounds of formula I in free form may be converted into salt form, and vice versa, in a conventional manner. The compounds in free or salt form can be obtained in the form of hydrates or solvates containing a solvent used for crystallisation. Compounds of formula I can be recovered from reaction mixtures and purified in a conventional manner. Isomers, such as enantiomers, may be obtained in a conventional manner, e.g. by fractional crystallisation or asymmetric synthesis from correspondingly asymmetrically substituted, e.g. optically active, starting materials.

Pharmaceutically acceptable salts of the compound of formula I may be acid addition salts, including those of inorganic acids, for example hydrohalic acids such as hydrofluoric acid, hydrochloric acid, hydrobromic acid or hydroiodic acid, nitric acid, sulfuric acid, phosphoric acid; and organic acids such as formic acid, acetic acid, propionic acid, butyric acid, benzoic acid, o-hydroxybenzoic acid, p-hydroxybenzoic acid, p-chlorobenzoic acid, diphenylacetic acid, triphenylacetic acid, 1-hydroxynaphthalene-2-carboxylic acid, 3-hydroxynaphthalene-2-carboxylic acid, aliphatic hydroxy acids such as lactic acid, citric acid, tartaric acid or malic acid, dicarboxylic acids such as fumaric acid, maleic acid or succinic acid, and sulfonic acids such as methanesulfonic acid or benzenesulfonic acid. These salts may be prepared from compounds of formula I by known salt-forming procedures. Pharmaceutically acceptable solvates are generally hydrates.

Pharmaceutically acceptable solvates in accordance with the invention include those wherein the solvent of crystallisation may be isotopically substituted e.g. D₂O, d₆-acetone or d₆-DMSO. Antibacterial agents employed in the present invention include a variety of agents, most preferably, agents directed against *Pseudomonas aeruginosa, Hemophilus influenzae, Burkholderia cepacia,* and major oral pathogens. Examples of antibiotics include agents from the β-lactam class, quinolone class, aminoglycoside class, macrolide class, tetracycline class, cationic peptide class and the like.

Preferred antibacterial agents from the beta-lactam class include, for example, ceftazidime, cefpirome, cefepime, cefoperazone, imipenem, meropenem, piperacillin, mezlocillin, ticarcillin, the combination of piperacillin and tazobactam, the combination of ticarcillin and clavulanic acid, and BMS-180680 (Bristol Myers and Squibb) including their pharmaceutically acceptable salts. Preferred agents from the quinolone class include, for example, norfloxacin, ciprofloxacin, ofloxacin, lomefloxacin, pefloxacin, rufloxacin, and sparfloxacin, including their pharmaceutically acceptable salts.

Representative compounds from the aminoglycoside class include, for example, tobramycin, amikacin, gentamicin, kanamycin, streptomycin, neomycin and netlimicin, including their pharmaceutically acceptable salts. Representative compounds from the macrolide class include erythromycin, clarithromycin, and azithromycin, including their pharmaceutically acceptable salts. Representative compounds from tetracycline class include tetracycline, oxytetracycline, doxycycline, and minocycline, including their pharmaceutically acceptable salts. Other antibacterial agents included in this invention are: IB-367 (Intrabiotics Pharm, Inc., Mountainview, California, USA), and daptomycin (Cubist Pharmaceuticals, Boston, USA) including their pharmaceutically acceptable salts.

An especially preferred antibacterial agent of the present invention is tobramycin, which is effective against *Pseudomonas aeruginosa.* That bacteria grows in the endobronchial space and is found in the sputum of infected individuals. During exacerbations of infection, such growth also occurs in the alveoli. The most common representative disease of bacterial *Pseudomonas aeruginosa* endobronchial infection is cystic fibrosis.

Cystic fibrosis (CF) is a common genetic disease that is characterized by the inflammation and progressive destruction of lung tissue. The debilitation of the lungs in CF patients is associated with accumulation of purulent sputum produced as a result of chronic endobronchial infections caused by *H. influenzae, Staphylococcus aureaus* and *Pseudomonas aeruginosa.* Nearly all individuals suffering from CF eventually die of respiratory failure. The advent of anti-pseudomonal antibiotic aminoglycosides such as tobramycin has decreased the mortality of CF patients and increased their life-span.

Tobramycin is an aminoglycoside specifically active against *Pseudomonas aeruginosa.* When delivered parenterally for a short period of time, it has been shown to successfully treat exacerbations which occur in patients with CF.

Also in accordance with the invention, a kit is provided that is suitable for use in treatment of an inflammatory, infective or obstructive airways disease in a patient in need of such treatment. Such a kit provides at least two separately packaged containers, a first container comprising an effective amount of at least one antibacterial agent according to the present invention, and a second container comprising a compound of formula I in free or pharmaceutically acceptable salt or solvate form. Either of the first or second container, preferably dispenses an aerosol spray. Alternatively, a container according to the invention may be a vial, blister pack, or other conventional means for holding a pharmaceutically active agent.

Administration of the medicament or pharmaceutical composition as hereinbefore described, i.e. with (A) and (B) n admixture or separate, is preferably by inhalation, i.e. (A) and (B) or the mixture thereof are in inhalable form.

The inhalable form of the medicament may be, for example, an atomizable composition such as an aerosol comprising the active ingredients, i.e. (A) and (B) separately or in admixture, in solution or dispersion in a propellant, or a nebulisable composition comprising a solution or dispersion of the active ingredient in an aqueous, organic or aqueous/organic medium. For example, the inhalable form of the medicament may be an aerosol comprising a mixture of (A) and (B) in solution or dispersion in a propellant. In another example, the inhalable form is a nebulizable composition comprising a dispersion of (A) and (B) in an aqueous, organic or aqueous/organic medium.

An aerosol composition suitable for use as the inhalable form of the medicament may comprise the active ingredient in solution or dispersion in a propellant, which may be chosen from any of the propellants known in the art. Suitable such propellants include hydrocarbons such as n-propane, n-butane or isobutane or mixtures of two or more such hydrocarbons, and halogen-substituted hydrocarbons, for example chlorine and/or fluorine-substituted methanes, ethanes, propanes, butanes, cyclopropanes or cyclobutanes, such as dichlorodifluoromethane (CFC-12), trichlorofluoromethane (CFC-11), 1,2-dichloro-1,1,2,2-tetrafluoroethane (CFC-114) or, particularly, 1,1,1,2-tetrafluoroethane (HFA-134a), 1,1,1,2,3,3,3-heptafluoropropane (HFA-227), difluorochloromethane (HCFC-22) or mixtures of two or more such halogen-substituted hydrocarbons.

Where the active ingredient is present in suspension in the propellant, i.e. where it is present in particulate form dispersed in the propellant, the aerosol composition may also contain a lubricant and a surfactant, which may be chosen from those lubricants and surfactants known in the art. Other suitable aerosol compositions include surfactant-free or substantially surfactant-free aerosol compositions. The aerosol composition may contain up to about 5% by weight, for example 0.0001 to 5%, 0.001 to 5%, 0.001 to 3%, 0.001 to 2%, 0.001 to 1%, 0.001 to 0.1 %, or 0.001 to 0.01%, but preferably 0.01 to 0.5% by weight of the active ingredient, based on the weight of the propellant. Where present, the lubricant and surfactant may be in an amount up to 5% and 0.5% respectively by weight of the aerosol composition. The aerosol composition may also contain a co-solvent such as ethanol in an amount up to 30% by weight of the composition, particularly for administration from a pressurised metered dose inhalation device. The aerosol composition may further contain a bulking agent, for example a sugar such as lactose, sucrose, dextrose, mannitol or sorbitol, in an amount, for example, of up to 20%, usually 0.001 to 1%, by weight of the composition.

In another embodiment of the invention, the inhalable form of the medicament is a dry powder, i.e. (A) and (B) are present in a dry powder comprising finely divided (A) and (B) optionally together with at least one particulate pharmaceutically acceptable carrier, which may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose, for example lactose monohydrate or anhydrous lactose. The dry powder may be contained as unit doses in capsules of, for example, gelatin or plastic, or in blisters (e.g. of aluminium or plastic), for use in a dry powder inhalation device, which may be a single dose or multiple dose device, preferably in dosage units of (A) and (B) together with the carrier in amounts to bring the total weight of powder per capsule to from 5 mg to 50 mg. Alternatively, the dry powder may be contained in a reservoir in a multi-dose dry powder inhalation (MDDPI) device adapted to deliver, for example, 3-25 mg of dry powder per actuation.

In the finely divided particulate form of the medicament, and in the aerosol composition where at least one of the active ingredients are present in particulate form, the active ingredient may have an average particle diameter of up to about 10 µm, for example 0.1 to 5 µm, preferably 1 to 5 µm. The particulate carrier, where present, generally has a maximum particle diameter up to 500 µm, preferably up to 400 µm, and conveniently has a mean particle diameter of 40 to 300 µm, e.g. 50 to 250 µm. The particle size of the active ingredient, and that of a particulate carrier where present in dry powder compositions, can be reduced to the desired level by conventional methods, for example by grinding in an air-jet mill, ball mill or vibrator mill, sieving, microprecipitation, spray-drying, lyophilisation or controlled crystallisation from conventional solvents or from supercritical media.

The inhalable medicament may be administered using an inhalation device suitable for the inhalable form, such devices being well known in the art. Accordingly, the invention also provides a pharmaceutical product comprising a medicament or pharmaceutical composition as hereinbefore described in inhalable form as hereinbefore described in association with one or more inhalation devices. In a further aspect, the invention provides an inhalation device, or a pack of two or more inhalation devices, containing a medicament or pharmaceutical composition as hereinbefore described in inhalable form as hereinbefore described.

Where the inhalable form of the active ingredient is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy.

For example, an aerosol composition may be administered from a coated can, for example as described in EP-A-0642992.

Where the inhalable form of the active ingredient is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a handheld nebulizer, sometimes referred to as a soft mist or soft spray inhaler, for example an electronically controlled device such as an AERx (Aradigm, US) or Aerodose (Aerogen), or a mechanical device such as a RESPIMAT (Boehringer Ingelheim) nebulizer which allows much smaller nebulised volumes, e.g. 10 to 100 µl, than conventional nebulisers.

Where the inhalable form of the active ingredient is the finely divided particulate form, the inhalation device may be, for example, a dry powder inhalation device adapted to deliver dry powder from a capsule or blister containing a dry powder comprising a dosage unit of (A) and/or (B) or a multi-dose dry powder inhalation (MDDPI) device adapted to deliver, for example, 3-25 mg of dry powder comprising a dosage unit of (A) and/or (B) per actuation. The dry powder composition preferably contains a diluent or carrier, such as lactose, and a compound that helps to protect against product performance deterioration due to moisture e.g. magnesium stearate, typically 0.05-2.0%. Suitable such dry powder inhalation devices are well known. For example, a suitable device for delivery of dry powder in encapsulated form is that described in US 3991761, while suitable MDDPI devices include those described in WO 97/20589 and WO 97/30743. Suitable devices that include means for deaggregating and aerosolising a dry powder are disclosed in US 6026809, US 6142146, US 6152130, WO 05/81833 and WO 05/81977.

The medicament of the invention is preferably a pharmaceutical composition comprising a mixture of (A) as hereinbefore defined and (B) as hereinbefore defined preferably together with at least one pharmaceutically acceptable carrier as hereinbefore described.

A suitable daily dose of the compound (A), the β₂-agonist, for inhalation may be from 20 µg to 2000 µg, for example from 20 to 1500 µg, from 20 to 1000 µg, preferably from 50 to 800 µg, e.g. from 100 to 600 µg or from 100 to 500 µg.

A suitable daily dose of (B) the antibacterial agent for inhalation may be from about 20 to about 500 mg, more preferably from about 40 to about 400 mg, and most preferably about 300 mg of the antibacterial agent per 5 ml of the quarter normal saline. For an aminoglycoside antibacterial agent this corresponds to about 4 to about 100 mg/ml, more preferably about 8 to about 80 mg/ml, and most preferably about 60 m/ml of the antibacterial agent, which is minimal yet efficacious amount of the antibacterial agent to suppress the *Pseudomonas aeruginosa* infections in endobronchial space. Typically, about 300 mg of the antibacterial agent is dissolved in 5 ml solution of a diluted, typically quarter normal saline containing about 0. 225% NaCl. Quarter normal saline, that is 0.225% of sodium chloride, is a presently preferred vehicle for delivery of the antibacterial agent into endobronchial space.

These unit doses may be administered once or twice daily in accordance with the daily doses mentioned hereinbefore. A single dose is preferred as this is convenient for the patient and encourages compliance. The precise doses of (A) and (B) used will of course depend on the condition to be treated, the patient and the efficiency of the inhalation device.

In one preferred embodiment of the invention, the medicament of the invention is a pharmaceutical composition which is a dry powder in a capsule containing unit doses of (A) and (B), for example for inhalation from a single capsule inhaler, the capsule suitably containing a unit dose of (A) e.g. as hereinbefore described, and a unit dose of (B), e.g. as hereinbefore described, together with a pharmaceutically acceptable carrier as hereinbefore described in an amount to bring the total weight of dry powder per capsule to between 5 mg and 50 mg, e.g. 5 mg, 10 mg, 15 mg, 20 mg, 25 mg, 30 mg, 35 mg, 40 mg, 45 mg or 50 mg.

In another preferred embodiment of the invention, the medicament of the invention is a pharmaceutical composition which is a dry powder for administration from a reservoir of a multi-dose dry powder inhaler adapted to deliver, for example, 3 mg to 25 mg of powder containing a unit dose of (A) and (B) per actuation.

In a further preferred embodiment of the invention, the medicament of the invention is a pharmaceutical composition which is an aerosol comprising (A) and (B) as hereinbefore described in a propellant as hereinbefore described, optionally together with a surfactant and/or a bulking agent and/or a co-solvent such as ethanol as hereinbefore described, for administration from a metered dose inhaler adapted to deliver an amount of aerosol containing a unit dose of (A) and a unit dose of (B), or a known fraction of a unit dose of (A) and a known fraction of a unit dose of (B) per actuation. Thus if, for example, the inhaler delivers half of the unit doses of (A) and (B) per actuation, the unit doses can be administered by two actuations of the inhaler.

In accordance with the above, the invention also provides a pharmaceutical kit comprising (A) and (B) as hereinbefore defined in separate unit dosage forms, said forms being suitable for administration of (A) and (B) in effective amounts. Such a kit suitably further comprises one or more inhalation devices for administration of (A) and (B). For example, the kit may comprise one or more dry powder inhalation devices adapted to deliver dry powder from a capsule, together with capsules containing a dry powder comprising a dosage unit of (A) and capsules containing a dry powder comprising a dosage unit of (B). In another example, the kit may comprise a multi-dose dry powder inhalation device containing in the reservoir thereof a dry powder comprising (A) and a multidose dry powder inhalation device containing in the reservoir thereof a dry powder comprising (B). In a yet further example, the kit may comprise a metered dose inhaler containing an aerosol comprising (A) in a propellant and a metered dose inhaler containing an aerosol comprising (B) in a propellant.

Medicaments of the invention are advantageous in the treatment of inflammatory, infective or obstructive airways diseases, exhibiting highly effective bronchodilatory and anti-inflammatory properties. Furthermore, using the combinations of the invention, particularly using compositions containing (A) and (B), medicaments which have a rapid onset of action and a long duration of action may be prepared. Moreover, using such combination therapy, medicaments which result in a significant improvement in lung function may be prepared. In another aspect, using the combination therapy of the invention, medicaments which provide effective control of inflammatory, infective or obstructive airways diseases, or a reduction in exacerbations of such diseases, may be prepared. In a further aspect, using compositions of the invention containing (A) and (B), medicaments which reduce or eliminate the need for treatment with short-acting rescue medicaments such as salbutamol or terbutaline, may be prepared; thus compositions of the invention containing (A) and (B) facilitate the treatment of an inflammatory, infective or obstructive airways disease with a single medicament.

Treatment of inflammatory, infective or obstructive airways diseases in accordance with the invention may be symptomatic or prophylactic treatment. Inflammatory, infective or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory, infective or obstructive airways diseases and conditions to which the present invention is applicable include acute lung injury (ALI), adult or acute respiratory distress syndrome (ARDS), cystic fibrosis, chronic obstructive pulmonary, airways or lung disease (COPD, COAD or COLD), including chronic bronchitis and emphysema, bronchiectasis and exacerbation of airways hyperreactivity consequent to other drug therapy, in particular other inhaled drug therapy. Further inflammatory, infective or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The medicament of the present invention may additionally contain one or more co-therapeutic agents such as anti-inflammatory, bronchodilatory, antihistamine, decongestant or anti-tussive drug substances, particularly in the treatment of inflammatory, infective or obstructive airways diseases such as those mentioned hereinbefore, for example as potentiators of therapeutic activity of such drugs or as a means of reducing required dosaging or potential side effects of such drugs.

Co-therapeutic agents include A_{2A} agonists, A_{2B} antagonists, antihistamines, antimuscarinic agents, caspase inhibitors, LTB4 antagonists, LTD4 antagonists, PDE4 inhibitors, steroids, mucolytics, matrix metal loproteinase inhibitors (MMPi's), leukotrienes, anti neoplastics, peptides, vaccines, elastase inhibitors and sodium cromoglycate including combinations thereof.

Suitable A_{2A} agonists include those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083.

Suitable A_{2B} antagonists include those described in WO 02/42298 and WO 03/042214.

Suitable antihistamine drug substances include cetirizine hydrochloride, levocetirizine, acetaminophen, clemastine fumarate, promethazine, loratidine, desloratidine, diphenhydramine and fexofenadine hydrochloride, activastine, astemizole, azelastine, dimetinden, ebastine, epinastine, levocabastine, mizolastine and tefenadine as well as those disclosed in WO 03/099807, WO 04/026841 and JP 2004107299.

Suitable antimuscarinic agents include ipratropium bromide, oxitropium bromide, tiotropium salts, CHF 4226 (Chiesi) and SVT-40776, or those described in EP 424021, US 3714357, US 5171744, US 2005/171147, US 2005/182091, WO 01/04118, WO 02/00652, WO 02/51841, WO 02/53564, WO 03/00840, WO 03/33495, WO 03/53966, WO 03/87094, WO 04/018422, WO 04/05285, WO 04/96800, WO 05/077361 and WO 06/48225. The medicament of the present invention optionally includes dual beta-2 adrenoceptor agonist / antimuscarinics such as those disclosed in US 2004/0167167, US 2004/0242622, US 2005/182092, US 2005/256114, US 2006/35933, WO 04/74246, WO 04/74812, WO 04/89892 and WO 06/23475.

Suitable caspase inhibitors, including interleukin- I P converting enzyme (ICE) inhibitors, include those that are disclosed in CA 2109646, GB 2,278,276EP 519748, EP 547 699, EP 590 650, EP 628550, EP 644 197, EP 644198, US 5411985, US 5416013, US 5430128, US 5434248, US 5565430, US 5585357, US 5656627, US 5677283, US 6054487, US 6531474, US 20030096737, WO 93/05071, WO 93/14777, WO 93/16710, WO 94/00154, WO 94/03480, WO 94/21673, WO 95/05152, WO 95/35308, WO 97/22618, WO 97/22619, WO 98/10778, WO 98/11109, WO 98/11129, WO 98/41232, WO 99/06367, WO 99/65451, WO 01/119373 and WO 03/32918.

Suitable LTB4 antagonists such as BIIL 284, CP-195543, DPC11870, LTB4 ethanolamide, LY 293111, LY 255283, CGS025019C, CP-195543, ONO-4057, SB 209247, SC-53228 and those described in US 5451700 and WO 04/108720.

Suitable LTD4 antagonists such as montelukast, pranlukast, zafirlukast, accolate, SR2640, Wy-48,252, ICI 198615, MK-571, LY-171883, Ro 24-5913 and L-648051.

Suitable PDE4 inhibitors PDE4 inhibitors such as cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), GRC 3886 (Oglemilast, Glenmark), and those described in WO 92/19594, WO 93/19749, WO 93/19750, WO 93/19751, WO 98/18796, WO 99/16766, WO 01/13953, WO 03/39544, WO 03/104204, WO 03/104205, WO 04/000814, WO 04/000839, WO 04/005258 , WO 04018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607, WO 04/037805, WO 04/063197, WO 04/103998, WO 04/111044, WO 05/012252, WO 05/012253, WO 05/013995, WO 05/030725, WO 05/030212, WO 05/087744, WO 05/087745, WO 05/087749 and WO 05/090345.

Suitable steroids include for example glucocorticosteroids such as budesonide, beclamethasone dipropionate, fluticasone propionate, mometasone furoate, ciclesonide, or steroids described in WO 02/00679, WO 02/88167, WO 02/12266, WO 02/100879, WO 03/35668, WO 03/48181, WO 03/62259, WO 03/64445, WO 03/72592, WO 04/39827 and WO 04/66920, or non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935, WO 04/26248 and WO 05/05452.

While (A) is a beta-2 adrenoceptor agonist, the medicament of the present invention optionally includes one or more other beta-2 adrenoceptor agonists such as include albuterol (salbutamol), metaproterenol, terbutaline, salmeterol, fenoterol, procaterol, and especially, formoterol, carmoterol, GSK159797 and pharmaceutically acceptable salts thereof, as well as those described in EP 147719, EP 1440966, EP 1460064, EP 1477167, EP 1574501, JP 05025045, JP 2005187357, US 2002/0055651, US 2004/0242622, US 2004/0229904, US 2005/0133417, US 2005/5159448, US 2005/5159448, US 2005/171147, US 2005/182091, US 2005/182092, US 2005/209227, US 2005/256115, US 2005/277632, US 2005/272769, US 2005/239778, US 2005/215542, US 2005/215590, US 2006/19991, US 2006/58530, WO 93/18007, WO 99/64035, WO 01/42193, WO 01/83462, WO 02/66422, WO 02/70490, WO 02/76933, WO 03/24439, WO 03/42160, WO 03/42164, WO 03/72539, WO 03/91204, WO 03/99764, WO 04/16578, WO 04/16601, WO 04/22547, WO 04/32921, WO 04/33412, WO 04/37768, WO 04/37773, WO 04/37807, WO 04/39762, WO 04/39766, WO 04/45618 WO 04/46083 , WO 04/80964, WO 04/087142, WO 04/89892, WO 04/108675, WO 04/108676, WO 05/33121, WO 05/40103, WO 05/44787, WO 05/58867, WO 05/65650, WO 05/66140, WO 05/70908, WO 05/74924, WO 05/77361, WO 05/90288, WO 05/92860, WO 05/92887, WO 05/90287, WO 05/95328, WO 05/102350, WO 06/56471, WO 06/74897 or WO 06/8173.

The invention is illustrated by the following Examples.

### EXAMPLES

### Compound A1

### (R)-5-[2-(5,6-diethyl-indan-2-ylamino)-1-hydroxy-ethyl]-8-hydroxy-1H-quinolin-2-one maleate

This compound is prepared by reacting (R)-8-benzyloxy-5-oxiranylcarbostyril with 5,6-diethyl-indan-2-ylamine to give 8-benzyloxy-5-[(R)-2-(5,6-diethyl-indan-2-ylamino)-1-hydroxy-ethyl]-IH-quinolin-2-one, subjecting the latter to a deprotecting reaction to replace the benzyl group by hydrogen, and recovering the resultant compound as a maleate salt. Such a process is described in detail in WO 2004/76422, the contents of which is incorporated herein by reference. (R)-8-benzyloxy-5-oxiranylcarbostyril may be prepared as described in W0 1995/25104. 5,6-Diethyl-indan-2-ylamine may be prepared as described in WO 2003/76387.

### Compound B1

Sub-MIC tobramycin is commercially available from Chiron as TOBI®.

### Examples 1-60

Gelatin capsules suitable for use in a capsule inhaler such as that described in US 3991761 and EP 1270034 are prepared, each capsule containing a dry powder obtained by mixing Compound A1 and Compound B1 which have been ground to a mean particle diameter of 1 to 5 µm and lactose monohydrate having a particle diameter below 212 µm, the amounts being as shown in the Table 1 below:

**TABLE 1**

| Example | **Compound A1** (Parts) | **Compound B1** (Parts) |
|---|---|---|
| 1 | 20 | 100 |
| 2 | 40 | 100 |
| 3 | 80 | 100 |
| 4 | 100 | 100 |
| 5 | 120 | 100 |
| 6 | 140 | 100 |
| 7 | 160 | 100 |
| 8 | 180 | 100 |
| 9 | 200 | 100 |
| 10 | 220 | 100 |
| 11 | 240 | 100 |
| 12 | 300 | 100 |
| 13 | 500 | 100 |
| 14 | 1000 | 100 |
| 15 | 2000 | 100 |
| 16 | 20 | 100 |
| 17 | 40 | 100 |
| 18 | 80 | 100 |
| 19 | 100 | 100 |
| 20 | 120 | 100 |
| 21 | 140 | 100 |
| 22 | 160 | 100 |
| 23 | 180 | 100 |
| 24 | 200 | 100 |
| 25 | 220 | 100 |
| 26 | 240 | 100 |
| 27 | 300 | 100 |
| 28 | 500 | 100 |
| 29 | 1000 | 100 |
| 30 | 2000 | 100 |
| 31 | 20 | 200 |
| 32 | 40 | 200 |
| 33 | 80 | 200 |
| 34 | 100 | 200 |
| 35 | 120 | 200 |
| 36 | 140 | 200 |
| 37 | 160 | 200 |
| 38 | 180 | 200 |
| 39 | 200 | 200 |
| 40 | 220 | 200 |
| 41 | 240 | 200 |
| 42 | 300 | 200 |
| 43 | 500 | 200 |
| 44 | 1000 | 200 |
| 45 | 2000 | 200 |
| 46 | 20 | 200 |
| 47 | 40 | 200 |
| 48 | 80 | 200 |
| 49 | 100 | 200 |
| 50 | 120 | 200 |
| 51 | 140 | 200 |
| 52 | 160 | 200 |
| 53 | 180 | 200 |
| 54 | 200 | 200 |
| 55 | 220 | 200 |
| 56 | 240 | 200 |
| 57 | 300 | 200 |
| 58 | 500 | 200 |
| 59 | 1000 | 200 |
| 60 | 2000 | 200 |

### Examples 61-105

A nebulised solution is prepared is by mixing Compound A1 and Compound B1 in a Pari® LC Plus jet nebulizer in 5 ml concentrated form in an aerosol producing a particle size having the mass medium average diameter predominately between 1 and 5 microlitres, the amounts being as shown in the Table 2 below:

**TABLE 2**

| Example | **Compound A1** (Parts) | **Compound B2** (Parts) |
|---|---|---|
| 61 | 20 | 100 |
| 62 | 40 | 100 |
| 63 | 80 | 100 |
| 64 | 100 | 100 |
| 65 | 120 | 100 |
| 66 | 140 | 100 |
| 67 | 160 | 100 |
| 68 | 180 | 100 |
| 69 | 200 | 100 |
| 70 | 220 | 100 |
| 71 | 240 | 100 |
| 72 | 300 | 100 |
| 73 | 500 | 100 |
| 74 | 1000 | 100 |
| 75 | 2000 | 100 |
| 76 | 20 | 200 |
| 77 | 40 | 200 |
| 78 | 80 | 200 |
| 79 | 100 | 200 |
| 80 | 120 | 200 |
| 81 | 140 | 200 |
| 82 | 160 | 200 |
| 83 | 180 | 200 |
| 84 | 200 | 200 |
| 85 | 220 | 200 |
| 86 | 240 | 200 |
| 87 | 300 | 200 |
| 88 | 500 | 200 |
| 89 | 1000 | 200 |
| 90 | 2000 | 200 |
| 91 | 20 | 250 |
| 92 | 40 | 250 |
| 93 | 80 | 250 |
| 94 | 100 | 250 |
| 95 | 120 | 250 |
| 96 | 140 | 250 |
| 97 | 160 | 250 |
| 98 | 180 | 250 |
| 99 | 200 | 250 |
| 100 | 220 | 250 |
| 101 | 240 | 250 |
| 102 | 300 | 250 |
| 103 | 500 | 250 |
| 104 | 1000 | 250 |
| 105 | 2000 | 250 |

## Claims

1. A medicament comprising, separately or together
(A) a compound of formula I 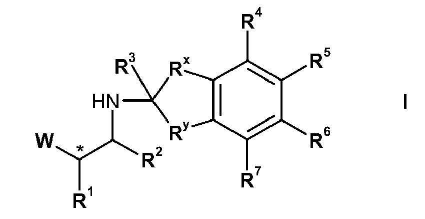 in free or salt or solvate form, wherein
W is a group of formula 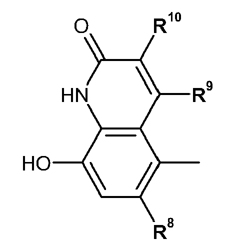 R^{x} and R^{y} are both -CH₂- or -(CH₂)₂-;
R¹ is hydrogen, hydroxy, or C₁-C₁₀-alkoxy;
R² and R³ are each independently hydrogen or C₁-C₁₀-alkyl;
R⁴, R⁵, R⁶ and R⁷ are each independently hydrogen, halogen, cyano, hydroxy,
C₁-C₁₀-alkoxy, C₆-C₁₀-aryl, C₁-C₁₀-alkyl, C₁-C₁₀-alkyl substituted by one or more halogen atoms or one or more hydroxy or C₁-C₁₀-alkoxy groups, C₁-C₁₀-alkyl interrupted by one or more hetero atoms, C₂-C₁₀-alkenyl, trialkylsilyl, carboxy, C₁-C₁₀-alkoxycarbonyl, or -CONR¹¹R¹² where R¹¹ and R¹² are each independently hydrogen or C₁-C₁₀-alkyl,
or R⁴ and R⁵, R⁵ and R⁶, or R⁶ and R⁷ together with the carbon atoms to which they are attached denote a 5-, 6- or 7-membered carbocyclic ring or a 4- to 10-membered heterocyclic ring; and
R⁸, R⁹ and R¹⁰ are each independently hydrogen or C₁-C₄-alkyl; and
(B) an antibacterial agent;
for simultaneous, sequential or separate administration in the treatment of an inflammatory, infective or obstructive airways disease.

2. A medicament according to claim 1 which is a pharmaceutical composition comprising a mixture of effective amounts of (A) and (B) optionally together with at least one pharmaceutically acceptable carrier.

3. A medicament according to claim 1 or 2, in which (A) is a compound of formula I in free or salt or solvate form wherein R⁸, R⁹ and R¹⁰ are each H, R¹ is OH, R² and R³ are each H and
(i) R^{x} and R^{y} are both -CH₂-, and R⁴ and R⁷ are each CH₃O- and R⁵ and R⁶ are each H;
(ii) R^{x} and R^{y} are both -CH₂-, and R⁴ and R⁷ are each H and R⁵ and R⁶ are each CH₃CH₂-;
(iii) R^{x} and R^{y} are both -CH₂-, and R⁴ and R⁷ are each H and R⁵ and R⁶ are each CH₃-;
(iv) R^{x} and R^{y} are both -CH₂-, and R⁴ and R⁷ are each CH₃CH₂- and R⁵ and R⁶ are each H;
(v) R^{x} and R^{y} are both -CH₂-, and R⁴ and R⁷ are each H and R⁵ and R⁶ together denote -(CH₂)₄-;
(vi) R^{x} and R^{y} are both -CH₂-, and R⁴ and R⁷ are each H and R⁵ and R⁶ together denote -O(CH₂)₂O-;
(vii) R^{x} and R^{y} are both -CH₂-, and R⁴ and R⁷ are each H and R⁵ and R⁶ are each CH₃(CH₂)₃-;
(viii) R^{x} and R^{y} are both -CH₂-, and R⁴ and R⁷ are each H and R⁵ and R⁶ are each CH₃(CH₂)₂-;
(ix) R^{x} and R^{y} are both -(CH₂)₂-, R⁴, R⁵, R⁶ and R⁷ are each H; or
(x) R^{x} and R^{y} are both -CH₂-, and R⁴ and R⁷ are each H and R⁵ and R⁶ are each CH₃OCH₂-.

4. A medicament according to any preceding claim, in which (A) is a compound of formula I selected from the group consisting of 8-hydroxy-5-[1-hydroxy-2-(indan-2-yl-amino)-ethyl]-1H-quinolin-2-one, 5-[2-(5,6-dimethoxy-indan-2-ylamino)-1-hydroxy-ethyl]-8-hydroxy-1H-quinolin-2-one, 5-[2-(5,6-diethyl-indan-2-ylamino)-1-hydroxy-ethyl]-8-hydroxy-3-methyl-1H-quinolin-2-one, 5-[2-(5,6-diethyl-indan-2-ylamino)-1-hydroxy-ethyl]-8-methoxy-methoxy-6-methyl-1H-quinolin-2-one, 5-[2-(5,6-diethyl-indan-2-ylamino)-1-hydroxy-ethyl]-8-hydroxy-6-methyl-1H-quinolin-2-one, 8-hydroxy-5-[2-(5,6-diethyl-indan-2-ylamino)-1-hydroxy-ethyl]-3,4-dihydro-1H-quinolin-2-one, 5-[(R)-2-(5,6-diethyl-2-methyl-indan-2-yl-amino)-1-hydroxyethyl]-8-hydroxy-1H-quinolin-2-one, (S)-5-[2-(4,7-diethyl-indan-2-ylamino)-1-hydroxy-ethyl]-8-hydroxy-1H-quinolin-2-one hydrochloride, 5-[(R)-1-hydroxy-2-(6,7,8,9-tetrahydro-5H-benzocyclohepten-7-ylamino)-ethyl]- 8-hydroxy-1H-quinolin-2-one hydrochloride, (R)-5-[2-(5,6-diethyl-indan-2-ylamino)-1-hydroxy-ethyl]-8-hydroxy-1H-quinolin-2-one maleate, (R)-5-[2-(5,6-diethyl-indan-2-ylamino)-1-hydroxy-ethyl]-8-hydroxy-1H-quinolin-2-one hydrochloride, (R)-8-hydroxy-5-[(S)-1-hydroxy-2-(4,5,6,7-tetramethyl-indan-2-ylamino)-ethyl]-1H-quinolin-2-one, 8-hydroxy-5-[(R)-1-hydroxy-2-(2-methyl-indan-2-ylamino)-ethyl]-1H-quinolin-2-one, 5-[2-(5,6-diethyl-indan-2-ylamino)-ethyl]-8-hydroxy-1H-quinolin-2-one, 8-hydroxy-5-[(R)-1-hydroxy-2-(2-methyl-2,3,5,6,7,8-hexahydro-1H-cyclo-penta[b]naphthalen-2-ylamino)-ethyl]-1H-quinolin-2-one and 5-[(S)-2-(2,3,5,6,7,8-hexahydro-1H-cyclopenta[b]naphthalen-2-ylamino)-1-hydroxy-ethyl]-8-hydroxy-1H-quinolin-2-one.

5. A medicament according to any preceding claim, in which (A) is (R)-5-[2-(5,6-diethyl-indan-2-ylamino)-1-hydroxyethyl]-8-hydroxy-1H-quinolin-2-one maleate.

6. A medicament according to any preceding claim wherein the antibacterial agent is selected from the group consisting of ceftazidime, cefpirome, cefepime, cefoperazone, imipenem, meropenem, piperacillin, mezlocillin, ticarcillin, the combination of piperacillin and tazobactam, the combination of ticarcillin and clavulanic acid, BMS-180680, norfloxacin, ciprofloxacin, ofloxacin, lornefloxacin, pefloxacin, rufloxacin, sparfloxacin, tobramycin, amikacin, gentamicin, netlimicin, erythromycin, clarithromycin, azithromycin, tetracycline, oxytetracycline, doxycycline, minocycline, IB-367, daptomycin, triclosan and pharmaceutically acceptable salts of any of said antibacterial agents.

7. A medicament according to claim 6 wherein the antibacterial agent is tobramycin.

8. A medicament according to any preceding claim, which is in inhalable form and is
(i) an aerosol comprising a mixture of (A) and (B) in solution or dispersion in a propellant;
(ii) a combination of an aerosol containing (A) in solution or dispersion in a propellant and an aerosol containing (B) in solution or dispersion in a propellant;
(iii) a nebulizable composition comprising a dispersion of (A) and (B) in an aqueous, organic or aqueous/organic medium; or
(iv) a combination of a dispersion of (A) in an aqueous, organic or aqueous/organic medium and a dispersion of (B) in an aqueous, organic or aqueous/organic medium.

9. A medicament according to any one of claims 1 to 10, in which (A) and (B) are present in inhalable form as a dry powder comprising finely divided (A) and (B) optionally together with at least one particulate pharmaceutically acceptable carrier.

10. A medicament according to claim 8 or 9, in which (A) and (B) have an average particle diameter up to 10 µm.

11. A medicament according to any one of claims 1 to 10, which is
a dry powder in a capsule, the capsule containing a unit dose of (A) and a unit dose of (B) and a pharmaceutically acceptable carrier in an amount to bring the total weight of dry powder per capsule to between 5 mg and 50 mg; or
an aerosol comprising (A) and (B) in a propellant, optionally together with a surfactant and/or a bulking agent and/or a co-solvent suitable for administration from a metered dose inhaler adapted to deliver an amount of aerosol containing a unit dose of (A) and a unit dose of (B), or a known fraction of a unit dose of (A) and a known fraction of a unit dose of (B), per actuation.

12. A medicament according to any preceding claim further comprising one or more additional drug substances selected from the group consisting of A_{2A} agonists, A_{2B} antagonists, antihistamines, antimuscarinic agents, caspase inhibitors, LTB4 antagonists, LTD4 antagonists, PDE4 inhibitors, steroids, mucolytics, matrix metal loproteinase inhibitors, leukotrienes, anti neoplastics, peptides, vaccines, elastase inhibitors and sodium cromoglycate.

13. The use of (A) and (B) as defined in any one of claims 1 to 10 in the preparation of a medicament for combination therapy by simultaneous, sequential or separate administration of (A) and (B) in the treatment of an inflammatory, infective or obstructive airways disease.

14. The use of (A) as defined in any one of claims 1 to 5 and (B) as defined in any one of claims 1, 6, and 7 in the preparation of a medicament for combination therapy by simultaneous, sequential or separate administration of (A) and (B) in the treatment of asthma, chronic obstructive pulmonary disease or cystic fibrosis.

15. A pharmaceutical kit comprising (A) as defined in any one of claims 1 to 5, (B) as defined in any one of claims 1, 6, and 7 in separate unit dosage forms, said forms being suitable for administration of (A) and (B) in effective amounts, together with one or more inhalation devices for administration of (A) and (B).
